# EUROPEAN PATENT SPECIFICATION

(11) **EP 4 233 745 B1**
(45) Date of publication and mention of the grant of the patent: **02.07.2025**
(21) Application number: 23174741.1
(22) Date of filing: 06.03.2018
(51) Int. Cl.: A61B 17/34

(54) **ENDOSCOPIC ULTRASOUND GUIDED ACCESS DEVICE**
ENDOSKOPISCHES ULTRASCHALLGEFÜHRTES ZUGANGSINSTRUMENT
DISPOSITIF D'ACCÈS ENDOSCOPIQUE GUIDÉ PAR ULTRASONS

(30) Priority: 07.03.2017 US 201762468210 P
(43) Date of publication of application: 30.08.2023
(62) Divisional of application: 18713480.4
(73) Proprietor: Boston Scientific Scimed, Inc., Maple Grove, MN 55311 (US)
(72) Inventor: BENNING, Christopher, Hopkinton, Massachusetts 01748 (US); GESSLER, Raymond, III, Roberts, Wisconsin 54023 (US); PHELAN, Jessica, Westford, Massachusetts 01886 (US); PALKHIWALA, Kushal, Chelmsford, Massachusetts 01824 (US); BANNON, Bryan, Duxbury, Massachusetts 02332 (US); DAYTON, Peter, Brookline, Massachusetts 02445 (US); WHITNEY, Andrew, Douglas, Massachusetts 01516 (US)
(74) Representative: Vossius & Partner Patentanwälte Rechtsanwälte mbB

(56) References cited:
- WO-A1-93/10837
- US-A- 5 403 311
- US-A1- 2016 015 421

## Description

### Priority Claim

The present disclosure claims priority to U.S. Provisional Patent Application Serial No. 62/468,210 filed March 3, 2017.

### Background

The pancreas and biliary system together form an important part of the digestive system. The pancreas and liver produce digestive fluids (pancreatic juice and bile) which help in the process of digestion (i.e., the breakdown of foods into parts which can be absorbed easily and used by the body). These digestive fluids are passed through the pancreatic duct and ducts of the biliary system prior to exiting into the intestine. Blockage of any of these ducts by, for example, a cancer, gallstone or scarring, may result in the duct becoming backed up and filled with fluid, requiring drainage.

US 2016/015421 A1 relates to a system for endoscopic ultrasound guided drainage which includes an access sheath extending longitudinally from a proximal end to a distal end and an access lumen extending therethrough from the proximal end to the distal end, a stylet slidably received within the access lumen, the stylet extending longitudinally from a proximal end to distal end and including a channel extending therethrough, the channel configured to receive a fluid therethrough, and a dilating sheath extending longitudinally from a proximal end to a distal end and including a dilating lumen extending therethrough. The dilating lumen is sized and shaped to slidably receive the access sheath.

US 5 403 311 A relates to a catheter which includes a flexible elongated catheter body that defines a needle-receiving, or probe-receiving, lumen, a retractable tissue-penetrable needle, or probe, and an electrode mounted on the distal portion of the catheter body. The needle provides a fluid passage for introducing fluid into tissue to permit the introduction of sclerotic agents for enhancing electrocoagulation of the tissue, heat-responsive drugs for improving the bonding to tissue surfaces, or vaso-constrictor drugs. The probe can also have a passage for fluid. The electrode can provide bipolar electro-coagulation of tissue in combination with an additional electrode mounted on the catheter body, or alternatively, the electrode can be employed in combination with either the needle or probe to establish a bipolar electro-coagulation path through tissue. The needle, or probe, in combination with an external electrode can be used to provide unipolar electro-coagulation, or ablation. In certain instances, the electrode can be used to provide a mapping function inside cardiac chambers.

WO 93/10837 A1 relates to a retractable safety penetrating instrument which includes a cannula and a needle disposed within the cannula and supported in a manner to automatically move proximally from an extended position wherein a sharp distal end of the needle protrudes from the cannula to a retracted position wherein the sharp distal end of the needle is protected in response to distal movement of the retractable safety penetrating instrument upon penetration into a cavity in the body. A retracting mechanism moves the needle proximally, is normally locked in a position preventing proximal movement of the needle and is released by distal movement of an operating member to trigger retraction of the needle.

### Summary

The invention is defined by the features of the independent claim. Further embodiments are disclosed in the dependent claims. All examples and embodiments not falling under the scope of the independent claim do not form part of the invention.

The present disclosure relates to a system for endoscopic ultrasound guided drainage comprising an access sheath including an elongated tube extending longitudinally from a proximal end to a distal end and including an access lumen extending therethrough from the proximal end to the distal end and a flexible tip coupled to the distal end of the elongated tube, the flexible tip biased to a curved configuration, a sharp slidably received within the access lumen, the sharp extending longitudinally from a proximal end to distal end and including a channel extending therethrough, the channel configured to receive a fluid therethrough, and a dilating sheath extending longitudinally from a proximal end to a distal end and including a dilating lumen extending therethrough, the dilating lumen sized and shaped to slidably receive the access sheath.

In an embodiment, the curved configuration of the flexible tip is a J-shape.

In an embodiment, the flexible tip is formed of a flexible polymeric material which permits the curved distal portion to be moved to a straightened configuration when the sharp is received therein.

In an embodiment, the access sheath is formed of a polymer coated metal coil to allow torque transmission in both the clockwise and counter clockwise direction.

In an embodiment, the access sheath includes laser cut sections for increased flexibility.

In an embodiment, the system includes a handle assembly coupled to a proximal end of each of the sharp, access sheath and dilating sheath.

In an embodiment, the handle assembly includes an actuator for moving the dilating sheath longitudinally relative to the access sheath.

In an embodiment, the handle assembly includes a generator connection coupled to the actuator.

In an embodiment, the dilating sheath including an insulated coil conductor at a distal end thereof configured to cauterize tissue.

In an embodiment, a distal portion of the sharp has a multi-facet puncture tip including holes to allow fluid to flow therethrough.

In an embodiment, the access sheath is fluoroscopically visible.

The present disclosure also relates to a system for endoscopic drainage comprising an access sheath extending longitudinally from a proximal end to a distal end and including an access lumen extending therethrough from the proximal end to the distal end, a sharp slidably received within the access lumen, the sharp extending longitudinally from a proximal end to distal tip and including a channel extending therethrough, the channel configured to receive a fluid therethrough, a dilating sheath extending longitudinally from a proximal end to a distal end and including a dilating lumen extending therethrough, the dilating lumen sized and shaped to slidably receive the access sheath, and a handle assembly including a sharp attachment mechanism coupled to a proximal end thereof, the sharp exiting the handle assembly via a proximal opening therein such that a proximal end of the sharp is coupled to the sharp attachment mechanism.

In an embodiment, the sharp attachment mechanism includes an injection port for injecting fluid into the channel of the sharp.

In an embodiment, the handle assembly includes an access sheath rotation knob at a proximal end thereof.

In an embodiment, the attachment mechanism is coupled to the handle assembly by one of a press fit, mechanical lock or friction fit.

The present disclosure also relates to a method for endoscopic ultrasound guided drainage comprising inserting an access sheath and a sharp through a working channel of an endoscope into a target duct within a body, the sharp extending through a lumen of the access sheath such that a distal tip of the sharp extends distally past a distal end of the access sheath so that the distal tip punctures the target duct, rotating the access sheath via a rotation knob at a proximal end thereof to adjust the direction of the sharp, injecting a contrast media through a channel of the sharp into the target duct to visually verify that the target duct is filled with fluids, and advancing a dilating sheath distally over the access sheath and into the target duct to dilate the target duct.

In an embodiment, the method further includes removing the sharp from the access sheath so that a distal portion of the access sheath reverts to a curved configuration.

In an embodiment, the method further includes dilating a puncture point in a surface of the target duct via an electrode of the dilating sheath.

In an embodiment, the electrode is an coil conductor.

In an embodiment, the method further includes cauterizing a surface of the target duct via a ceramic dilating sheath tip.

### Brief Description of the Drawings

Fig. 1 shows a longitudinal cross-sectional view of a system according to an exemplary embodiment of the present disclosure;
Fig. 2 shows a longitudinal partial cross-sectional view of a distal portion of a sharp assembly according to the system of claim 1;
Fig. 3 shows a longitudinal partial cross-sectional view of a distal portion of a sharp assembly according to a second exemplary embodiment of the present disclosure;
Fig. 4 shows a longitudinal cross-sectional view of an access sheath according to the system of Fig. 1;
Fig. 5 shows a side view of a portion of the access sheath according to another exemplary embodiment of the present disclosure;
Fig. 6 shows a cross-sectional view of a dilating sheath according to an exemplary embodiment of the present disclosure;
Fig. 7 shows a perspective view of a handle assembly of the system of Fig. 1;
Figs. 8A and 8B show side views of a sharp attachment mechanism of the system of Fig. 1 according to a first exemplary embodiment;
Fig. 9 shows a side view of a sharp attachment mechanism according to a second exemplary embodiment;
Fig. 10 shows a side view of a sharp attachment mechanism according to a third exemplary embodiment; and
Figs. 11A, 11B and 11C show a side, top and side views, respectively, of a sharp attachment mechanism according to a fourth exemplary embodiment.

### Detailed Description

The present disclosure may be further understood with reference to the following description and the appended drawings, wherein like elements are referred to with the same reference numerals. The present disclosure is directed to endoscopic medical devices and, in particular, relate to endoscopic ultrasound (EUS) guided drainage. Exemplary embodiments describe a EUS guided drainage systems comprising a sharp for injecting a fluid into a fluid-filled duct, an access sheath through which the sharp is inserted and a dilating sheath for dilating the fluid-filled duct to facilitate drainage. It will be understood by those of skill in the art that the system and method of the present disclosure may be used to drain, for example, a bile duct, a pancreatic duct, cysts, gallbladder, etc. It should be noted that the terms "proximal" and "distal" as used herein are intended to refer to a direction toward (proximal) and away from (distal) a user of the device.

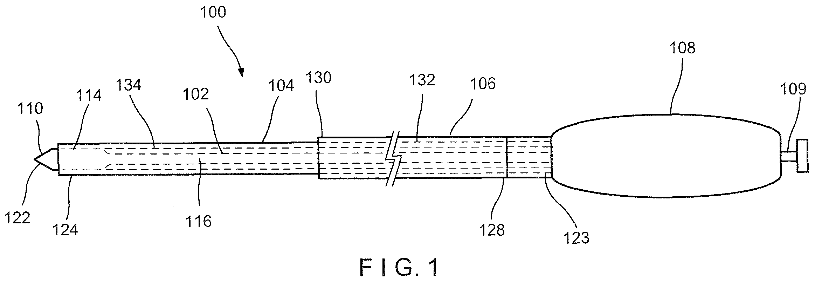

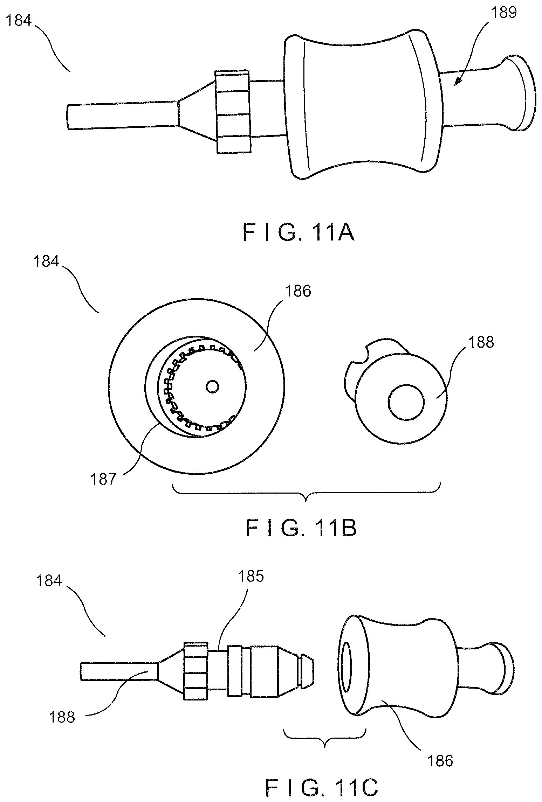

As shown in Figs. 1 - 11C, a system 100 according to an exemplary embodiment of the present disclosure comprises a sharp 102 for puncturing a fluid-filled tract and injecting a fluid (e.g., contrast media) thereinto and an access sheath 104 for providing access into the fluid-filled tract. The system 100 further comprises a dilating sheath 106 for dilating the tract to facilitate drainage. The system 100 is sized and shaped to be passed through a working channel of an endoscope to be visualized under ultrasound guidance. The system 100 may further comprise a handle assembly 108, which remains outside of a living body while the sharp 102 and the access sheath 104 are inserted therein (e.g., along a tortuous path through a natural body lumen accessed via a naturally occurring body orifice). The handle assembly 108 permits the sharp 102 to be removed therefrom while the access sheath 104 remains in the target duct. The handle assembly 108 also includes an actuator for advancing the access sheath 104 beyond the dilating sheath 106 and another actuator for advancing the dilating sheath 106 over the access sheath 104 and into the target duct.

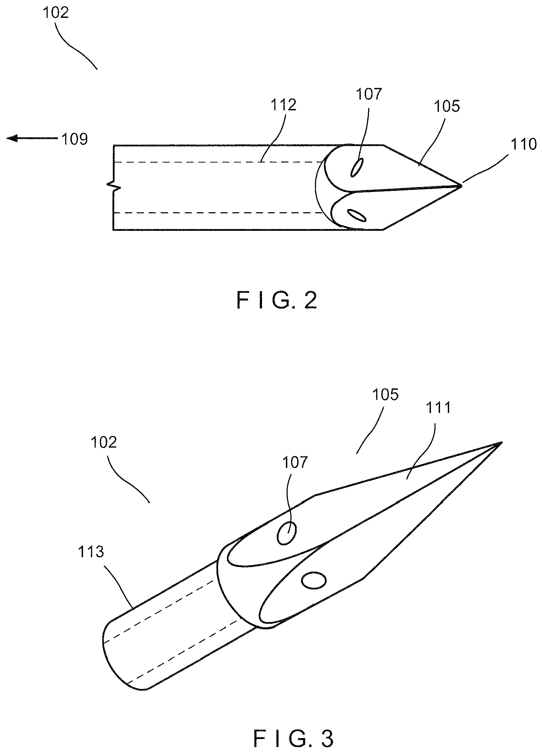

As shown in Figs. 2-3, a sharp 102 extends along a longitudinal axis from a proximal end 109 to a distal end 110 and includes a channel 112 extending therethrough. The sharp 102 may be formed from Nitinol, Stainless Steel or any variety of bio-compatible metals with a similar stiffness. In an alternate embodiment, the sharp 102 may be formed of plastic or another suitable polymer. In an embodiment, the sharp 102 may be formed with a flexbile design such as, for example, a coil or a spiral cut design. The sharp 102 may be configured as a hypotube with a multi-facet distal tip 105 at a distal end thereof for puncturing the target duct. The tip 105 according to this embodiment include holes 107 open to the channel 112 to allow fluid injection (e.g. contrast media) from the proximal end through the channel 112 to exit the distal end of the device. In an exemplary embodiment, the tip 105 may be attached to the hypotube via welding, bonding, or mechanical fastening such as threads. In an alternate embodiment, the hypotube 103 and tip 105 may be formed of a plastic or any other suitable polymer. In this embodiment, the plastic tip 105 may be constructed from one or two components. In the single body design, the tip 105 may be constructed, for example, through molding. In the double component design, a distal tapered portion 111 may be attached to a base tube 113, as seen in Fig. 3, via adhesive, melting or heat shrink application. The channel 112 extends from the proximal end 109 of the sharp 102 along the longitudinal axis thereof to a distal end 110 extending through the tip 105. In another exemplary embodiment, the puncture sharp 102 may be formed as a hypotube with either a sharpened wall or beveled edge along the circumference of the distal leading edge to promote puncture and allow a large opening for fluid injection. In this embodiment, the channel 112 extends from a proximal portion 116 of the sharp 102 and is open at the distal end 110 of the sharp 102. A fluid such as, for example, contrast media, may be injected into the target duct via the channel 112 to verify that the target duct is filled with fluid (e.g., digestive fluid).

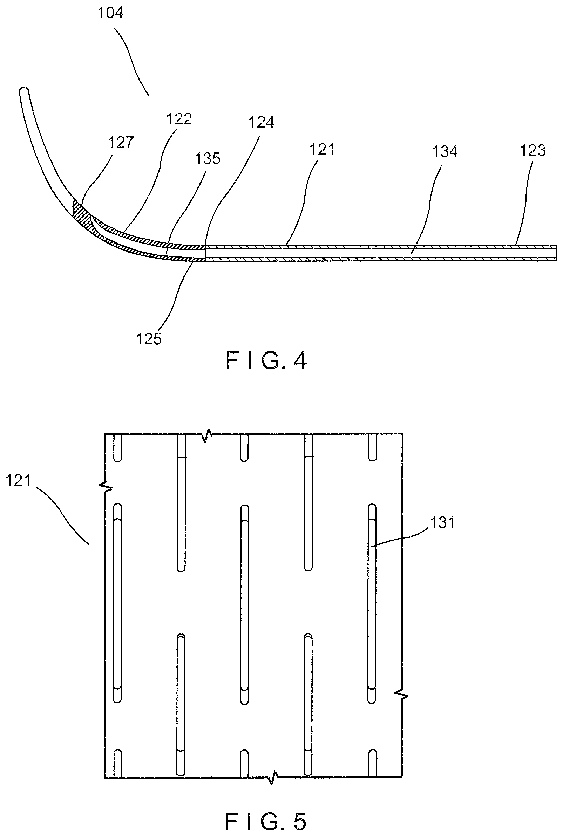

As shown in Fig. 4, the access sheath 104 includes a hollow tube 121 and a flexible tip 122. The hollow tube 121 extends longitudinally from a proximal end 123 to a distal end 124 and includes a lumen 134 extending therethrough. The lumen 134 is sized and shaped so that the sharp 102 can slight through. In particular, an inner diameter of the lumen 134 in this embodiment substantially corresponds to an outer diameter of the sharp 102 so that when the sharp 102 is received therein it completely fills the lumen 134 of the hollow tube 121. Flexible tip 122 extends from a proximal end 125 to a distal end 127 and includes a lumen 135 extending therethrough. Similar to the hollow tube lumen 134, the flexible tip lumen 135 is sized and shaped to slidably receive the sharp 102 therein and may have an inner diameter substantially equal to the inner diameter of the hollow tube 121. In particular, an inner diameter of the flexible tip lumen 135 in this embodiment substantially corresponds to an outer diameter of the sharp 102 so that when the sharp 102 is received therein it completely fills the lumen 134 of the flexible tip 122 to facilitate puncturing the target duct when the access sheath 104, with the sharp 102 received therein, is inserted into the target duct. Furthermore, the flexible tip 122 of this embodiment may be biased to assume, when not constrained, a desired a curvature along a distal portion 127 thereof to direct the inserted sharp 102 and a guidewire toward a target site. In one exemplary embodiment, the distal portion 127 of the flexible tip 122 is biased toward a J configuration (i.e., a curve in which the distal portion 127 arcs away from an axis of more proximal portions of the sheath 104 along an arc of 90° or less) for directing a guidewire in another desired direction. The distal end 127 of the flexible tip 122 may have a taper or a rounded edge to minimize initial puncture forces and ensure the flexible tip 122 follows the sharp tip 105 into the target area. The proximal end 125 of the flexible tip 122 is coupled to the distal end 124 of the hollow tube 121 such that the hollow tube lumen 134 is aligned with and open to the lumen 135 of the flexible tip 122.

The flexible tip 122 may be formed of a polymer that is sufficiently flexible so that when the sharp 102 is received therein, the distal portion of the flexible tip122 is straightened. Once the sharp 102 is extended distally therefrom, however, the flexible tip 122 is permitted to revert to its curved configuration. The curved configuration is maintained when a distal floppy end of the guidewire is within the flexible tip 122. The hollow tube 121 and the flexible tip 122 are formed of different materials. In an exemplary embodiment, the access sheath 104 is formed of braid reinforced polyamide. In another embodiment, the access sheath 104 is formed of multiple polymeric layers such as multilayer braid constructions. In a further embodiment, the access sheath 104 is insulated or coated along its length or at portions thereof. For example, the hollow tube 121 may be formed of PTFE, ETFE, or other polymer coated single-wire or dual-wire-counter-wound metal coils that allow transmission of torque in both clockwise and counter clockwise direction in 1-to-1 ratio. The torque transmission permits the user to rotate and direct the guidewire with the formed tip toward a target site and the coating allows for compatibility with electrosurgical activation as will be discussed in more detail below. The coating also reduces friction and promotes electrosurgical compatibility with the metal used to form the hollow tube 121. It will be understood that insulation or coating is only required on portions of the access sheath 104 that could come into contact with the operator or patient. In another exemplary embodiment, the hollow tube 121 is formed as a parylene or a similar polymer coated solid or laser cut hypotube 121 that allows transmission of torque in both the clockwise and counter-clockwise directions in a 1-to-1 ratio. In an embodiment, the solid hypotube 121 is made of Nitinol and has an inner diameter of 0.9271 +/- 0.0127 mm (0.0365 +/-0.0005 inches) and an outer diameter of 1.1049 +/- 0.0127 mm (0.0435 +/- 0.0005 inches). In another embodiment, the hypotube 121 has laser cut sections that increase flexibility in the hypotube. An exemplary laser cut design can be seen in Fig. 5 with cuts 131 tapered over the length of the device. The cuts 131 are concentrated in certain areas where increased flexibility is needed due to aspects of the procedure and the tortuosity of the path along which the scope extends. The parylene coating reduces friction and promotes electrosurgical compatibility with the metal used to form the hollow tube 121. The access sheath 104 assembly including the hollow tube 121 and the flexible tip 122 is fluoroscopy and EUS compatible. For example, the flexible tip 122 polymer may be loaded with Bismuth or Tungsten. In another example, marker bands may be added to the flexible tip 122 to facilitate visual determination of the position and orientation of the device. In a further example, echogenic features may be added to the hollow tube 121 to enhance ultrasonic imaging of the device as would be understood by those skilled in the art.

The dilating sheath 106 similarly extends longitudinally from a proximal end 128 to a distal end 130 and includes a lumen 132 extending therethrough and a distal portion 131. The lumen 132 is sized and shaped to slidably receive the access sheath 104 therein so that the dilating sheath 106 may be advanced over the access sheath 104 to the target duct to dilate the obstructed duct, thereby facilitating drainage thereof. The dilating sheath 106 may be a cold dilator such as, for example, a sohendra type dilator and/or a balloon dilator. Alternatively, the dilating sheath 106 may be a hot dilator such as, for example, a cystome or needleknife, which includes electrosurgical capabilities. For example, the dilating sheath 106 may include an electrode 137 extending along the distal portion 131 (immediately adjacent the distal end 130) thereof for cauterizing tissue. In particular, the dilating sheath 106 may be configured to utilize electrosurgical dissection to facilitate dilation or to burn a lesion as the dilating sheath 106 is inserted into the target duct. For example, the electrode may be an insulated coil conductor that is exposed at a distal end to supply cut/cautery energy. In another example, the distal portion 131 of the dilating sheath may be formed as a a tip (not shown) made from ceramic or another material with either a wire wrapped around the base or a gold-based painted on pattern extending to the distal end 130 of the sheath. It will be understood that the pattern may, in other examples, be any suitable material such as platinum, silver, titanium, stainless steel, niobium, titanium nitride, tungsten, copper or graphite-based inks. The tip may be configured as a cone, dome or any of a variety of configurations facilitating insertion into the target duct. In another embodiment, using "cold" dilation, the dilating sheath 106 may have a balloon (not shown) attached to the distal end 130. The balloon may be used in conjunction with previous tip designs or by itself. The balloon may be connected to a pump that inflates/deflates the balloon once it is in position. Once the dilating sheath 106 has been advanced over the access sheath 104 and inserted into the target duct, the dilating sheath 106 may be actuated to dilate or expand the target duct. For example, the dilating sheath 106 may have one or more stepped diameters at discrete distances from the distal end or one or more additional sheaths that may be independently actuated to expand the path to the target duct. The dilating sheath 106 may be fluoroscopically and EUS compatible. That is, the properties of the tip and the electrode provide visibility which aids with dilation of the access region.

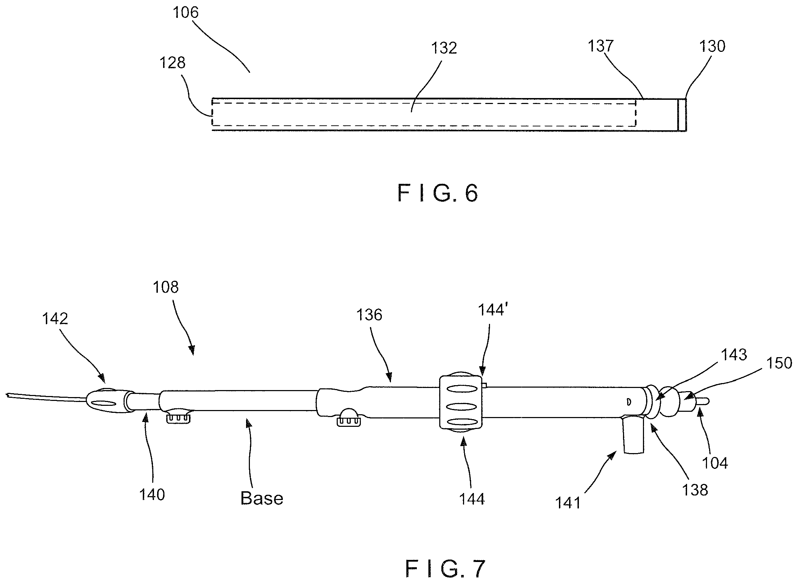

As shown in Fig. 7, the handle assembly 108 includes a grip portion 136 extending from a proximal end 138 to a distal end 140 and an extension portion 142 coupled to the distal end 140 of the grip portion 136 and couplable to the proximal end 128 of the dilating sheath 106. The access sheath 104 may be received within and coupled to the grip portion 136 such that the access sheath 104 extends through the lumen 132 of the dilating sheath 106. The sharp 102 extends through the grip portion 136 and the extension portion 142 with the proximal end of the sharp 102 extending proximally of the proximal end 138 of the grip portion and the length of the sharp 102 extending through the lumen 134 of the access sheath 104. Since the proximal end 109 of the sharp 102 extends proximally from the grip portion 136, the sharp 102 may be removed from the access sheath 104 by simply pulling the sharp 102 proximally relative to the handle assembly 108. The distal end 110 of the sharp 102 extends distally past the distal end 124 of the access sheath 104 so that the tapered tip 122 may puncture the target duct once the system 100 has been inserted into the body. The handle assembly 108 also includes an actuator 144 which moves the dilating sheath 106 longitudinally relative to the access sheath 104. In particular, the actuator 144 may include a tab that is moved distally and proximally with respect to the grip portion 136 of the handle assembly 108 to advance and retract, respectively, the dilating sheath 106 over the access sheath 104. The handle assembly 108 may include an access sheath lock 144' which locks how far the access sheath 104 extends beyond the dilating sheath 106. The handle may also include a dilating sheath lock (not shown) which locks the dilating sheath 106 in a specific location. In use, the access sheath lock may be unlocked while the access sheath/style assembly is through into the target tissue beyond the dilating sheath tip, then the dilating sheath 106 is unlocked and the dilating sheath 106 is advanced over the access sheath 106. As noted above, in embodiments in which the dilating sheath 106 includes an electrode, an active wire (not shown) is used to provide a current to the electrode. In a preferred embodiment, the active wire is long enough to run the length of the device in the extended position (before any cautery or puncture activation) and the contracted position (puncture and cautery activated). In the current embodiment, the wire may be coiled around the access sheath 104 in the handle assembly 108. Coiling the wire prevents it from kinking during handle actuation and while permitting the wire to have a length sufficient for both the extended and contracted handle configurations. In an alternate embodiment, the handle assembly 108 may include a generator connection 141 located at a proximal portion thereof. The generator connection 141 may be attached to the actuator 144 and may move with the actuator during actuation. Thus, in this embodiment, slack management of the active wire 139 would not be required. The handle assembly 108 further includes an access sheath rotation knob 143 located at a proximal end thereof. The rotation knob 143 is coupled to the proximal end of the access sheath 104 via an access sheath hub (not shown) at the handle and is rotatable, allowing transmission of torque in both the clockwise and counter clockwise direction in a 1-to-1 ratio.

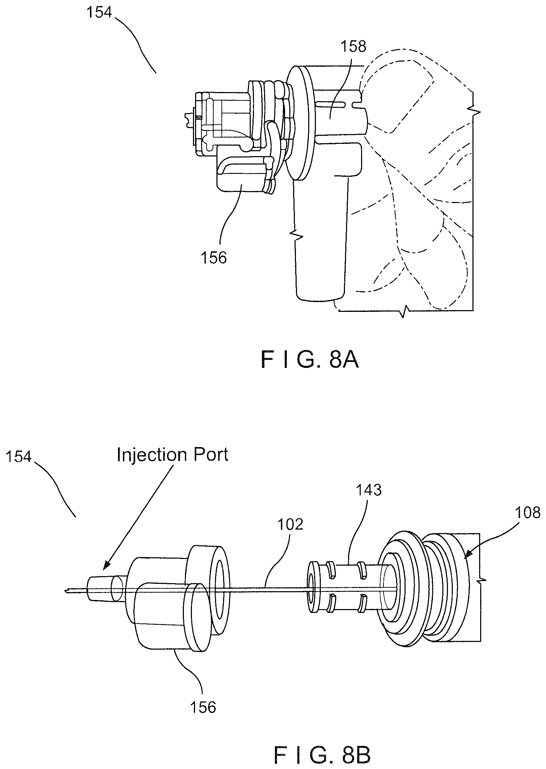

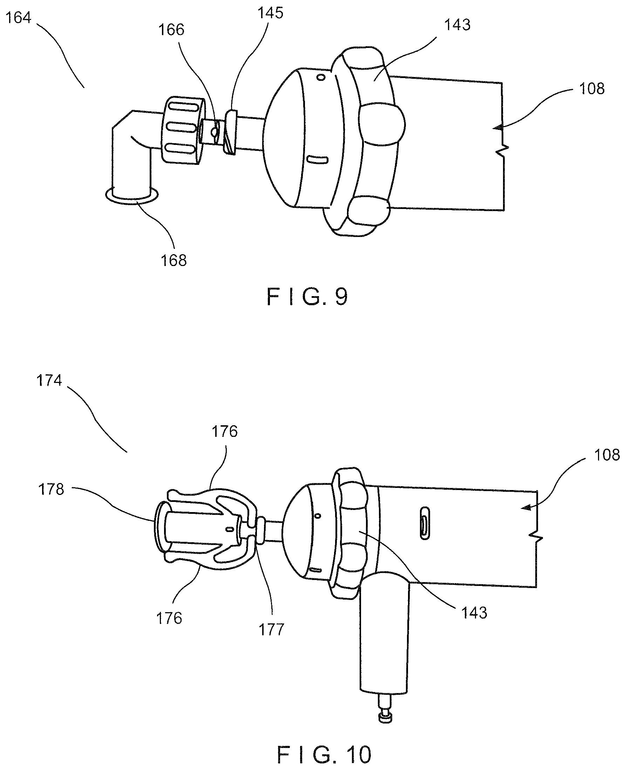

The handle assembly 108 includes a sharp attachment mechanism 150 which allows the sharp 102 to be easily attached to, and removed from, the handle assembly 108. The sharp 102 extends proximally from a proximal end of the handle assembly 108, with a proximal end 109 thereof coupled to the sharp attachment mechanism 150. The sharp attachment mechanism 150 also allows for fluid to be injected into the sharp channel 112 through an injection port 152. It is noted that in an exemplary embodiment, the injection port 152 may be added to the access sheath 104 to allow injection through the access sheath lumen. A first exemplary sharp attachment mechanism 150 uses a "top hat" design as seen in Figs. 8A and 8B. This "top hat" mechanism 154 utilizes a mechanical side lock 156 to attach the sharp 102 to the handle assembly 108. When side lock 156 is depressed, an inner lumen (not shown) of the lock 156 aligns with a handle top feature 158, unlocking the sharp 102 from the handle assembly 108. The "top hat" attachment mechanism 154 also engages an inner portion of the rotation knob 143 via the side lock 156 to facilitate rotation of the access sheath 104. Another exemplary embodiment of the sharp attachment mechanism 150 uses a "press fit" design as seen in Fig. 9. The "press fit" attachment 164 uses friction fit to attach the sharp 102 to the handle assembly 108. The 90-degree component 166 uses a friction fit to hold the sharp within a lumen 145 of the access sheath rotation knob 143. Similar to the "top hap" design 154, the 90-degree component 166 of the "press fit" design 164 includes a side port 168 for injection of fluid into the sharp channel 112. Another exemplary attachment mechanism 150 uses a "harp" design as seen in Fig. 10. This design utilizes a mechanical lock to attach the sharp 102 to the handle 108. The "harp" attachment 174 includes two side wings 176 which may be pressed simultaneously inward so that a clamp 177 that holds the sharp 102 to the handle 108 is released, allowing the sharp 102 to be retracted into the handle assembly 108. The "harp" attachment 174 includes an injection port 178 at its proximal end. A further exemplary embodiment of the sharp attachment mechanism 150 uses a "snap cap" design as seen in Figs. 11A-11C. The "snap cap" attachment 184 includes a press fit lock to attach the sharp 102 to the handle 108. The attachment includes a proximal half portion 186 and a distal half portion188, with the distal half portion 188 secured in the handle assembly 108. The proximal half portion 186 is removably attached to the distal half portion 188 via a locking slot 185 extending about the diameter of the bottom half portion 188. The proximal half portion 186 includes a coil portion 187 that moves over the distal half portion 188 of the design and sits in the locking slot 185 when the two components are attached. Similar to the "top hat" and "harp" attachments 154, 174, the "snap cap" attachment 184 includes an injection port at its proximal end 189.

According to a method using the system 100 according to an exemplary embodiment of the present disclosure, the system 100 is inserted through a working channel of an endoscope via, for example, ultrasound guidance to a target duct within the body. In an insertion configuration, the access sheath 104 according to an exemplary embodiment is fully housed within the dilating sheath 106 to protect an interior surface of a working channel of an endoscope or other insertion device through which the system 100 is inserted from the sharp distal tip 122 of the sharp 102. Upon insertion through the endoscope, the dilating sheath 106 may be in a proximal position so that the dilating sheath 106 does not extend distally over the portion of the access sheath 104 being inserted into the target duct. At this point, the distal end 110 of the sharp 102 extends distally past the distal end 124 of the access sheath 104. The access sheath 104 and sharp 102 is then advanced distally to penetrate the target duct. Once the sharp 102 and the access sheath 104 have been inserted into the target duct, contrast media (e.g., radiopaque dye) may be inserted, via the injection port 152, through the channel 112 of the sharp 102, out of the holes 107 of the sharp tip 105 into the target duct so that a user of the system 100 may visually verify that the duct has been filled with fluid and requires drainage. The sharp 102 may then be removed from the access sheath 104 by drawing the sharp 102 proximally relative to the access sheath 104 so that only the access sheath 104 remains in the target duct. Upon removal of the sharp 102, the flexible tip 122 of the access sheath 104 is freed to revert to the curved configuration to either anchor the access sheath 104 in the target duct or to direct a guidewire therethrough in a desired direction. If the access sheath 104 is in the target duct, a guidewire may be inserted through the lumen 134 of the access sheath 104 and into the target duct. As would be understood by those skilled in the art, a tip of the guidewire passed through the access sheath 104 will be directed in a direction corresponding to a curvature of the distal portion 126 of the access sheath 104 to contact an interior surface of the target duct. Rotation of the handle may then be used to manipulate the position of the j-shape, thus allowing the operator to advance the guidewire in a chosen direction. It will be understood that direction may or may not be set before guidewire advancement. As would be understood by those skilled in the art, prior to inserting the guide wire into the access sheath 104, the access sheath 104 may be rotated by manipulating the access sheath rotation knob 143 to direct the curved flexible tip 122 toward a desired direction.

Once the access sheath 104 has been anchored in the target duct, the dilating sheath 106 may be advanced over the access sheath 104 into the target duct. At this point, the generator connection 141 may be connected to a surgical generator such as, for example, a high-frequency (HF), alternating current (AC) surgical generator to provide an active current to the wire 139. As described above, the dilating sheath 106 is advanced by moving the actuator 144 distally with respect to the grip portion 136 of the handle assembly 108. The distal end 130 of the dilating sheath 106 is configured to facilitate insertion of the dilating sheath 106 into the target duct and to be advanced to a site at which the duct is blocked. In one embodiment, an electrode at the distal end 130 is activated to electrosurgically dissect and/or cauterize a surface tissue of the target duct to facilitate insertion therein. Insertion of the dilating sheath 106 over the site of the blockage enlarges the portion of the duct surround the obstruction to permit drainage of the target duct. It will be understood by those of skill in the art that the dilating sheath 106 may dilate the target duct in any of a number of ways. In one previously described example, the dilating sheath 106 includes an expansible balloon activated to expand the target duct. It will be understood by those of skill in the art that a user may also implement further treatment of the blocked duct. In particular, a stent may be implanted in the duct at the location of the blockage to maintain the duct in an enlarged configuration to ensure continued drainage thereof.

It will be apparent to those skilled in the art that various modifications may be made in the present disclosure, without departing from the scope of the disclosure. Thus, it is intended that the present disclosure cover the modifications and variations of his disclosure provided that they come within the scope of the appended claims.

## Claims

1. A system for endoscopic ultrasound guided drainage, comprising:
an access sheath (104) including an elongated tube (121) extending longitudinally from a proximal end (123) to a distal end (124) and including an access lumen (134) extending therethrough from the proximal end to the distal end and a flexible tip (122) coupled to the distal end of the elongated tube, the flexible tip biased toward a curved configuration;
a sharp (102) slidably received within the access lumen, the sharp extending longitudinally from a proximal end (109) to a distal end (110) and including a fluid channel (112) extending therethrough to at least one opening (107) in a distal end of the sharp, the sharp being configured so that, when the sharp is received within the distal end of the access sheath, the flexible tip of the access sheath is straightened; and
a dilating sheath (106) extending longitudinally from a proximal end to a distal end and including a dilating lumen (132) extending therethrough, the dilating lumen sized and shaped to slidably receive the access sheath,
wherein the elongated tube (121) and the flexible tip (122) are formed of different materials.

2. The system of claim 1, wherein the curved configuration of the flexible tip (122) is a J-shape; and/or wherein the flexible tip is formed of a flexible polymeric material.

3. The system of claim 1 or claim 2, wherein the access sheath (104) is formed of a polymer coated metal coil to allow torque transmission in both the clockwise and counter clockwise direction.

4. The system of any one of claims 1 to 3, wherein at least a portion of the access sheath (104) is formed of a thin-walled hypotube wherein the hypotube includes at least one laser cut section having an increased flexibility relative to a section of the hypotube that does not include laser cuts.

5. The system of claim 1, further comprising a handle assembly (108) coupled to a proximal end of each of the access sheath (104) and the dilating sheath (106), wherein the handle assembly includes a first actuator for moving the access sheath assembly relative to the dilating sheath and a second actuator (144) for moving the dilating sheath longitudinally relative to the access sheath, preferably wherein the handle assembly includes a generator connection (141) coupled to the actuator.

6. The system of claim 5, further comprising an access sheath rotation knob (143) coupled to the handle assembly (108) and coupled to a proximal end of the access sheath (104) so that rotation of the access sheath rotation knob rotates the access sheath about a longitudinal axis of the access sheath.

7. The system of any one of claims 1 to 6, wherein a distal portion of the sharp (102) has a multi-facet puncture tip (105) and wherein the opening (107) is formed in the multi-facet puncture tip to allow fluid to flow therethrough.

8. The system of any one of claims 1 to 7, wherein the access sheath (104) is formed of a material selected to be fluoroscopically visible.

9. The system of claim 5, wherein the handle assembly (108) includes a sharp attachment mechanism (150) coupled to a proximal end thereof, the sharp (102) exiting the handle assembly via a proximal opening the handle such that a proximal end (109) of the sharp is coupled to the sharp attachment mechanism, preferably wherein the sharp attachment mechanism includes an injection port for injecting fluid into the channel (112) of the sharp.

10. The system of claim 9, wherein the sharp attachment mechanism (150) is coupled to the handle assembly (108) by one of a press fit, mechanical lock or friction fit.

11. The system of claim 1, wherein the access sheath (104) includes at least one marker and/or echogenic feature to enhance visualization

12. The system of claim 1, wherein the dilating sheath (106) includes an electrode (137) at a distal end thereof for cauterizing tissue.

13. The system of claim 12, wherein the electrode (137) is formed as a coil conductor exposed on an exterior of the dilating sheath (106).

14. The system of claim 1, wherein the access sheath (104) is movable longitudinally within the dilating sheath (106) and wherein the handle assembly (108) includes an access sheath lock (144') that is actuatable to lock a position of the access sheath relative to the dilating sheath.

15. The system of claim 9, wherein the sharp attachment mechanism (150) is configured to be coupled to a proximal end of the handle assembly (108).

## Patentansprüche

1. System zur endoskopischen ultraschallgeführten Drainage, das aufweist:
ein Zugangshüllrohr (104) mit einer länglichen Röhre (121), die sich von einem proximalen Ende (123) zu einem distalen Ende (124) längs erstreckt und ein Zugangslumen (134), das sich durch sie hindurch vom proximalen Ende zum distalen Ende erstreckt, und eine flexible Spitze (122) aufweist, die mit dem distalen Ende der länglichen Röhre gekoppelt ist, wobei die flexible Spitze in eine gekrümmte Konfiguration vorgespannt ist;
ein scharfkantiges Teil (102), das im Zugangslumen verschiebbar aufgenommen ist, wobei sich das scharfkantige Teil von einem proximalen Ende (109) zu einem distalen Ende (110) längs erstreckt und einen Fluidkanal (112) aufweist, der sich durch dieses hindurch zu mindestens einer Öffnung (107) in einem distalen Ende des scharfkantigen Teils erstreckt, wobei das scharfkantige Teil so konfiguriert ist, dass bei Aufnahme des scharfkantigen Teils im distalen Ende des Zugangshüllrohrs die flexible Spitze des Zugangshüllrohrs gerade gerichtet ist; und
eine Dilatationshülse (106), die sich von einem proximalen Ende zu einem distalen Ende längs erstreckt und ein sich durch sie hindurch erstreckendes Dilatationslumen (132) aufweist, wobei das Dilatationslumen so dimensioniert und geformt ist, dass es das Zugangshüllrohr verschiebbar aufnimmt,
wobei die längliche Röhre (121) und die flexible Spitze (122) aus unterschiedlichen Materialien gebildet sind.

2. System nach Anspruch 1, wobei die gekrümmte Konfiguration der flexiblen Spitze (122) J-förmig ist; und/oder wobei die flexible Spitze aus einem flexiblen Polymermaterial gebildet ist.

3. System nach Anspruch 1 oder Anspruch 2, wobei das Zugangshüllrohr (104) aus einer polymerbeschichteten Metallspirale gebildet ist, damit ein Drehmoment sowohl im Uhrzeigersinn als auch gegen den Uhrzeigersinn übertragen werden kann.

4. System nach einem der Ansprüche 1 bis 3, wobei mindestens ein Abschnitt des Zugangshüllrohrs (104) aus einem dünnwandigen Hypotubus gebildet ist, wobei der Hypotubus mindestens ein lasergeschnittenes Teilstück mit einer erhöhten Flexibilität relativ zu einem Teilstück des Hypotubus aufweist, das keine Laserschnitte aufweist.

5. System nach Anspruch 1, ferner mit einer Griffanordnung (108), die mit einem proximalen Ende jeweils des Zugangshüllrohrs (104) und der Dilatationshülse (106) gekoppelt ist, wobei die Griffanordnung einen ersten Aktor zum Bewegen der Zugangshüllrohranordnung relativ zur Dilatationshülse und einen zweiten Aktor (144) zum Längsbewegen der Dilatationshülse relativ zum Zugangshüllrohr aufweist, wobei die Griffanordnung vorzugsweise eine mit dem Aktor gekoppelte Generatorverbindung (141) aufweist.

6. System nach Anspruch 5, ferner mit einem Zugangshüllrohr-Drehknopf (143), der mit der Griffanordnung (108) gekoppelt und mit einem proximalen Ende des Zugangshüllrohrs (104) gekoppelt ist, so dass eine Drehung des Zugangshüllrohr-Drehknopfs das Zugangshüllrohr um eine Längsachse des Zugangshüllrohrs dreht.

7. System nach einem der Ansprüche 1 bis 6, wobei ein distaler Abschnitt des scharfkantigen Teils (102) eine mehrflächige Punktionsspitze (105) hat und wobei die Öffnung (107) in der mehrflächigen Punktionsspitze ausgebildet ist, damit Fluid durchströmen kann.

8. System nach einem der Ansprüche 1 bis 7, wobei das Zugangshüllrohr (104) aus einem Material gebildet ist, das so ausgewählt ist, dass es fluoroskopisch sichtbar ist.

9. System nach Anspruch 5, wobei die Griffanordnung (108) einen mit einem proximalen Ende von dieser gekoppelten Befestigungsmechanismus (150) für das scharfkantige Teil aufweist, wobei das scharfkantige Teil (102) aus der Griffanordnung über eine proximale Öffnung des Griffs austritt, so dass ein proximales Ende (109) des scharfkantigen Teils mit dem Befestigungsmechanismus für das scharfkantige Teil gekoppelt ist, wobei der Befestigungsmechanismus für das scharfkantige Teil vorzugsweise einen Injektionsanschluss zum Injizieren von Fluid in den Kanal (112) des scharfkantigen Teils aufweist.

10. System nach Anspruch 9, wobei der Befestigungsmechanismus (150) für das scharfkantige Teil mit der Griffanordnung (108) durch Presspassung, mechanische Verriegelung oder Reibpassung gekoppelt ist.

11. System nach Anspruch 1, wobei das Zugangshüllrohr (104) mindestens einen Marker und/oder ein echogenes Merkmal aufweist, um die Visualisierung zu verstärken.

12. System nach Anspruch 1, wobei die Dilatationshülse (106) eine Elektrode (137) an einem distalen Ende davon zum Kauterisieren von Gewebe aufweist.

13. System nach Anspruch 12, wobei die Elektrode (137) als Spiralleiter ausgebildet ist, der auf einer Außenseite der Dilatationshülse (106) freiliegt.

14. System nach Anspruch 1, wobei das Zugangshüllrohr (104) in der Dilatationshülse (106) längs beweglich ist und wobei die Griffanordnung (108) eine Zugangshüllrohrverriegelung (144') aufweist, die so betätigbar ist, dass sie eine Position des Zugangshüllrohrs relativ zur Dilatationshülse verriegelt.

15. System nach Anspruch 9, wobei der Befestigungsmechanismus (150) für das scharfkantige Teil so konfiguriert ist, dass er mit einem proximalen Ende der Griffanordnung (108) gekoppelt ist.

## Revendications

1. Système pour le drainage endoscopique guidé par ultrasons, comprenant :
une gaine d'accès (104) comprenant un tube allongé (121) s'étendant longitudinalement d'une extrémité proximale (123) à une extrémité distale (124) et comprenant une lumière d'accès (134) s'étendant à travers ce dernier, de l'extrémité proximale à l'extrémité distale et une pointe souple (122) couplée à l'extrémité distale du tube allongé, la pointe souple étant sollicitée dans une configuration courbe ;
un objet tranchant (102) reçu de manière coulissante dans la lumière d'accès, l'objet tranchant s'étendant longitudinalement d'une extrémité proximale (109) à une extrémité distale (110) et comprenant un canal de fluide (112) s'étendant à travers ce dernier jusqu'à au moins une ouverture (107) dans une extrémité distale de l'objet tranchant, l'objet tranchant étant configuré de sorte que, lorsque l'objet tranchant est reçu dans l'extrémité distale de la gaine d'accès, la pointe souple de la gaine d'accès est redressée ; et
une gaine de dilatation (106) s'étendant longitudinalement à partir d'une extrémité proximale jusqu'à une extrémité distale et comprenant une lumière de dilatation (132) s'étendant à travers cette dernière, la lumière de dilatation étant dimensionnée et formée pour recevoir, de manière coulissante, la gaine d'accès,
dans lequel le tube allongé (121) et la pointe souple (122) sont formés avec différents matériaux.

2. Système selon la revendication 1, dans lequel la configuration courbe de la pointe souple (122) est une forme de J ; et/ou la pointe souple est formée avec un matériau polymère souple.

3. Système selon la revendication 1 ou la revendication 2, dans lequel la gaine d'accès (104) est formée avec une bobine métallique recouverte de polymère pour permettre la transmission de couple à la fois dans le sens des aiguilles d'une montre et dans le sens inverse des aiguilles d'une montre.

4. Système selon l'une quelconque des revendications 1 à 3, dans lequel au moins une partie de la gaine d'accès (104) est formée avec hypotube à fine paroi, dans lequel l'hypotube comprend au moins une section découpée au laser présentant une flexibilité accrue par rapport à une section de l'hypotube qui ne comprend pas de découpes au laser.

5. Système selon la revendication 1, comprenant en outre un ensemble de poignée (108) couplé à une extrémité proximale de chacune parmi la gaine d'accès (104) et la gaine de dilatation (106), dans lequel l'ensemble de poignée comprend un premier actionneur pour déplacer l'ensemble de gaine d'accès par rapport à la gaine de dilatation, et un deuxième actionneur (144) pour déplacer la gaine de dilatation longitudinalement par rapport à la gaine d'accès, de préférence dans lequel l'ensemble de poignée comprend un raccordement de générateur (141) couplé à l'actionneur.

6. Système selon la revendication 5, comprenant en outre un bouton de rotation de gaine d'accès (143) couplé à l'ensemble de poignée (108) et couplé à une extrémité proximale de la gaine d'accès (104) de sorte que la rotation du bouton de rotation de gaine d'accès fait tourner la gaine d'accès autour d'un axe longitudinal de la gaine d'accès.

7. Système selon l'une quelconque des revendications 1 à 6, dans lequel une partie distale de l'objet tranchant (102) a une pointe de perforation à plusieurs surfaces (105) et dans lequel l'ouverture (107) est formée dans la pointe de perforation à plusieurs surfaces pour permettre que le fluide s'écoule à travers cette dernière.

8. Système selon l'une quelconque des revendications 1 à 7, dans lequel la gaine d'accès (104) est formée avec un matériau sélectionné pour être visible par voie fluoroscopique.

9. Système selon la revendication 5, dans lequel l'ensemble de poignée (108) comprend un mécanisme de fixation d'objet tranchant (150) couplé à son extrémité proximale, l'objet tranchant (102) sortant de l'ensemble de poignée via une ouverture proximale de la poignée, de sorte qu'une extrémité proximale (109) de l'objet tranchant est couplée au mécanisme de fixation d'objet tranchant, de préférence dans lequel le mécanisme de fixation d'objet tranchant comprend un orifice d'injection pour injecter du fluide dans le canal (112) de l'objet tranchant.

10. Système selon la revendication 9, dans lequel le mécanisme de fixation d'objet tranchant (150) est couplé à l'ensemble de poignée (108) par l'un parmi un ajustement serré, un verrouillage mécanique ou un ajustement par friction.

11. Système selon la revendication 1, dans lequel la gaine d'accès (104) comprend au moins un marqueur et/ou caractéristique échogène pour améliorer la visualisation.

12. Système selon la revendication 1, dans lequel la gaine de dilatation (106) comprend une électrode (137) au niveau de son extrémité distale pour cautériser le tissu.

13. Système selon la revendication 12, dans lequel l'électrode (137) est formée comme un conducteur hélicoïdal exposé sur un extérieur de la gaine de dilatation (106).

14. Système selon la revendication 1, dans lequel la gaine d'accès (104) est mobile longitudinalement dans la gaine de dilatation (106) et dans lequel l'ensemble de poignée (108) comprend un verrouillage de gaine d'accès (144') qui peut être actionné pour verrouiller une position de la gaine d'accès par rapport à la gaine de dilatation.

15. Système selon la revendication 9, dans lequel le mécanisme de fixation d'objet tranchant (150) est configuré pour être couplé à une extrémité proximale de l'ensemble de poignée (108).
